# FASCICULE DE BREVET EUROPEEN

(11) **EP 2 205 203 B1**
(45) Date de publication et mention de la délivrance du brevet: **23.01.2013**
(21) Numéro de dépôt: 08838707.1
(22) Date de dépôt: 30.09.2008
(51) Int. Cl.: C11D 1/72, C08G 65/30, C08G 65/18, C07C 43/11, B01F 17/00, C11D 1/722, C07C 43/13

(54) **NOUVEAUX COMPOSES PREPARES PAR ADDITION D'UN DERIVE OXETANE SUR UN ALCOOL**
DURCH ZUGABE EINES OXETANDERIVATS ZU ALKOHOL HERGESTELLTE NEUE VERBINDUNGEN
NOVEL COMPOUNDS PREPARED BY ADDING AN OXETANE DERIVATIVE TO AN ALCOHOL

(30) Priorité: 15.10.2007 FR 0758306
(43) Date de publication de la demande: 14.07.2010
(73) Titulaire: CECALC, 92700 COLOMBES (FR)
(72) Inventeur: DA COSTA, Georges, F-81710 Saix (FR); GUILBOT, Jérôme, F-81100 Castres (FR); MULLER, Daniel, F-94100 Saint Maur (FR); ROLLAND, Hervé, F-81100 Castres (FR)
(74) Mandataire: Lhoste, Catherine
(86) Numéro de dépôt international: PCT/FR2008/051751
(87) Numéro de publication internationale: WO 2009/050405

(56) Documents cités:
- EP-A- 0 779 289
- EP-A- 1 060 740
- WO-A-00/27903
- WO-A-01/14300
- FR-A- 2 804 432
- US-A- 2 854 486
- A. FISHMAN ET AL: "Synthesis and Investigation of Novel Branched PEG-Based Soluble Polymer Supports" J. ORG. CHEM., vol. 68, no. 25, 12 décembre 2003 (2003-12-12), pages 9843-9846, XP002480985
- EVANS R ET AL: "SOME ETHERS OF PENTAERYTHRITOL AND THEIR NITRATE ESTERS" JOURNAL OF THE AMERICAN CHEMICAL SOCIETY, AMERICAN CHEMICAL SOCIETY, WASHINGTON, DC, US, vol. 75, 1 mars 1953 (1953-03-01), page 1248/1249, XP002053798 ISSN: 0002-7863
- S. SEARLES ET AL: "Reactions of Alcohols and Phenols with Trimethylene Oxide" J.AM.CHEM.SOC., vol. 76, 1954, pages 56-58, XP002480986 cité dans la demande

## Description

L'invention a pour objet de nouvelles compositions obtenues à partir d'une réaction d'un alcool de nature variable, pouvant être modifié préalablement par addition d'oxyde d'alkylène, et d'un substrat se caractérisant par un motif oxétane et par au moins une fonction hydroxyle, le procédé de préparation de ces compositions et leur utilisation en tant qu'agents tensioactifs non ioniques.

Un agent tensioactif est une substance ou une composition chimique qui, même utilisé en faible quantité, réduit de façon importante la tension superficielle, en particulier celle de l'eau, ou la tension de surface entre deux liquides non miscibles de façon à faciliter les mélanges de ces deux liquides.

Par ailleurs, la structure amphiphile des agents tensioactifs leur confère une affinité particulière pour les interfaces de type air/eau et eau/huile et donc, par là même, leur donne la capacité d'abaisser l'énergie libre de ces surfaces. Ce phénomène est à la base de la stabilisation des systèmes dispersés.

Dans la publication : « Reactions of alcohols and phenols with trimethylene oxide », S. Searles, C.F. Butler, JACS, 1954, Vol. 76, P.56-58, l'ouverture du 1, 3 propylène oxyde ou oxétane non substitué par différents alcools en présence d'un catalyseur acide ou basique : 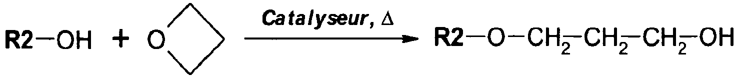 est décrite.

Néanmoins, la liste des alcools étudiés est restreinte à des alcools ne comprenant pas de chaîne alkyle susceptible de conférer un caractère amphiphile ou tensioactif à la structure des adduits finaux (méthanol, éthanol, propanol, butanol, isopropanol, alcool benzylique, phénol alcools) et l'utilisation d'oxétane non hydroxylé ne permet pas d'augmenter la fonctionnalité des adduits finaux et donc d'aboutir à des structures dont la polarité est convenablement prononcée.

La demande de brevet WO 01/14300 décrit un procédé de fabrication d'éther alcools à partir d'un oxétane hydroxylé et d'un polyol dérivé du 1,3-propanediol monosubstitué ou disubstitué, les stoechiométries molaires employées mettent systématiquement en jeu un défaut d'oxétane hydroxylé ne permettant pas la démultiplication des fonctions hydroxyle.

La demande de brevet EP 1060740 décrit la préparation des polyoléthers ou polyolhydroxyéthers par réaction du triméthylolpropane, du triméthylolbutane, du pentaérythritol ou du dipentaérythritol avec des alcools gras saturés ou insaturés ou des époxydes gras saturés ou insaturés, la dite préparation ne faisant pas intervenir un dérivé de type oxétane au niveau des réactifs.

Dans le cadre de leurs recherches sur la mise au point de nouveaux agents tensioactifs, les inventeurs ont développé de nouvelles structures issues de la condensation de synthons d'oxétane avec des dérivés d'alcools gras.

C'est pourquoi selon un premier aspect, l'invention a pour objet un composé de formule (I) : 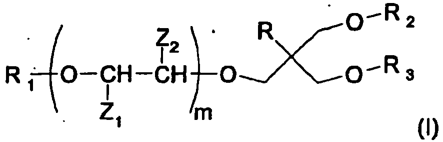 dans laquelle :
- R₁ représente un radical aliphatique saturé ou insaturé, linéaire ou ramifié, comportant de 7 à 24 atomes de carbone, éventuellement substitué avec un ou plusieurs groupes hydroxyle,
- R représente un radical alkyle linéaire ou ramifié comportant de un à huit atomes de carbone éventuellement substitué par un radical hydroxyle,
- m représente un nombre supérieur ou égal à 0 et inférieur ou égal à 150,
- Z₁ et Z₂ identiques ou différents sont choisis parmi H, CH₃, CH₂-CH₃,
- R₂ et R₃ identiques ou différents représentent soit un atome d'hydrogène,
soit un radical monovalent de formule : 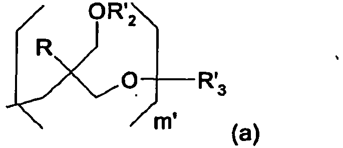 dans laquelle :
- m' représente un nombre inférieur ou égal à 10,
- R'₂ représente soit un atome d'hydrogène, soit un radical monovalent de formule : 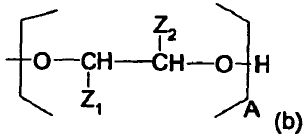 dans laquelle :
   ■ Z₁ et Z₂ sont tels que définis ci-dessus,
   ■ A représente un nombre entier supérieur ou égal à 0 et inférieur ou égal à 50,
- R'₃ identique à R'₂ ou différent de R'₂ représente soit un atome d'hydrogène, soit un radical monovalent de formule : 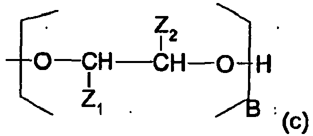 dans laquelle :
   ■ Z₁ et Z₂ sont tels que définis ci-dessus,
   ■ B représente un nombre entier supérieur ou égal à 0 et inférieur ou égal à 50,
avec A + B supérieur ou égal à 0 et inférieur ou égal à 50 et m + A + B ≠ 0.

De préférence, A + B est inférieur ou égal à 30 et plus particulièrement inférieur ou égal à 10. Avantageusement, m représente un nombre inférieur ou égal à 50. De préférence, m + A + B est inférieur ou égal à 50 et plus particulièrement inférieur ou égal à 30.

Selon un mode particulier de l'invention, m, A et B représentent chacun un nombre inférieur ou égal à 10.

Une composition comprenant un mélange de composés de formule (I) est également un objet de la présente invention.

Selon un aspect de l'invention, dans la formule (I) telle que définie précédemment, R'₂ et R'₃ sont des atomes d'hydrogène, dans ce cas précis, la formule (I) est alors notée (I₁) : 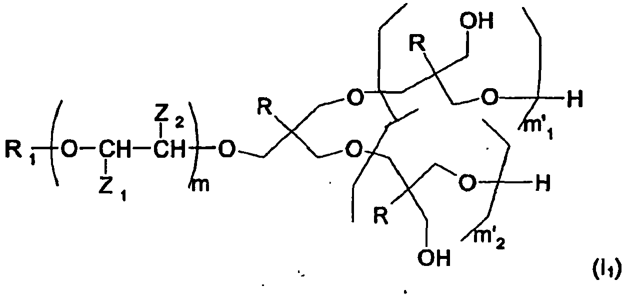 dans laquelle m'1 et m'2 représentent des nombres supérieurs ou égaux à 0 et Inférieurs ou égaux à 10.

Selon la présente invention, dans la formule (I) telle que définie précédemment R₂ représente un atome d'hydrogène et R₃ représente un radical de formule (a') : 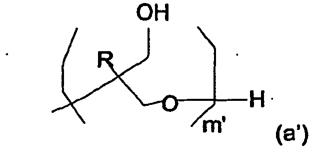 correspondant à la formule (a) dans laquelle R'₂ et R'₃ représentent chacun un atome d'hydrogène, cette formule sera notée (Io).

Selon un autre aspect de l'invention, dans la formule (I) telle que définie précédemment, R₂ et R₃ représentent un radical de formule (a') : 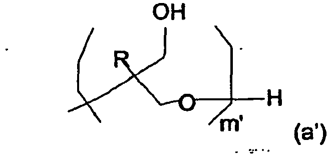 correspondant à la formule (a) dans laquelle R'₂ et R'₃ représentent chacun un atome d'hydrogène, cette formule sera notée (I'o).

Selon un autre aspect de la présente invention, dans la formule (I) telle que définie précédemment, R₂ ou R₃ n'est pas l'hydrogène.

Dans la formule (I), A peut indifféremment être égal à B ou différent de B.

Par radical alkyle linéaire ou ramifié comportant de un à huit atomes de carbone éventuellement substitué par un radical hydroxyle, on désigne pour R dans les formules définies ci-dessus : les radicaux méthyle, éthyle, propyle, iso-propyle, butyle, isobutyle, tertiobutyle, pentyle, néopentyle, hexyle, heptyle, octyle, methylol.

Par alcool de nature variable, on entend des alcools gras linéaires hydrocarbonés saturés ou insaturés présentant un nombre d'atomes de carbone allant de C-8 à C-24 (**R₁**). Ces alcools pourront également être des alcools ramifiés présentant un nombre d'atomes de carbone allant de C-8 à C-36 comme par exemple l'alcool isostéarylique, les alcools issus du procédé de synthèse « oxo », les alcools de Guerbet, les dimerdiols ou alcools provenant de l'hydrogénation de l'acide dimère ayant 36 atomes de carbone notamment commercialisés par les sociétés COGNIS ou SIDOBRE-SINNOVA sous la dénomination SPEZIOL C 36/2, ou être fonctionnalisés par un ou plusieurs motifs hydroxyle tel que l'alcool hydroxystéarylique par exemple.

Par dérivé oxétane présentant au moins une fonction hydroxyle, on entend les substrats suivants : triméthyloléthane- (**R** = Me), triméthylolpropane- (**R** = Et), triméthylolbutane-oxétane (**R** = Pr), triméthylolpentane- oxétane (**R** = Bu), triméthylolhexane-oxétane (**R** = -C₅H₁₁), triméthylolheptane-oxétane (**R** = -C₆H₁₃), triméthyloloctane- (**R** = -C₇H₁₅), triméthylolnonane-oxétane (**R** = -C₈H₁₇), pentaérythritol- oxétane (R = -CH₂OH).

L'invention a aussi pour objet un procédé de préparation d'un composé de formule (I₁) telle que définie précédemment, comprenant l'étape :
- (a) réaction d'au moins un alcool de formule: 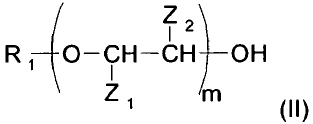 dans laquelle R1, Z₁ et Z₂ sont tels que définis dans la formule (I) ;
   et d'au moins un substrat, comportant un motif oxétane et au moins une fonction hydroxyle, de formule: 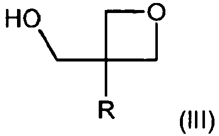
dans laquelle R est tel que défini dans la formule (I).

Lors de cette étape (a), l'ouverture du motif oxétane s'accompagne systématiquement de la formation d'une nouvelle fonction hydroxyle permettant ainsi d'augmenter progressivement la fonctionnalité des agents tensioactifs finaux par rapport à l'alcool de départ. A titre d'exemple, un adduit avec trois motifs oxétane ouverts présentera 4 fonctions hydroxyle, comme représenté par l'exemple de structure ci-dessous : 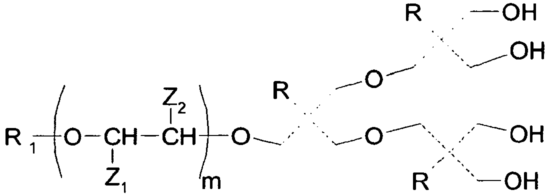 et plus généralement n motifs oxétane conduiront à n+1 fonctions hydroxyle. Cette configuration peut alors conduire, selon la stoechiométrie molaire en dérivé oxétane de départ, à des distributions de structures pseudo-dendritiques.

La présence de ces fonctions hydroxyle confère donc aux produits une hydrophilie plus ou moins prononcée et liée au nombre d'équivalents de dérivé oxétane additionnés ainsi qu'au caractère lipophile des alcools de départ utilisés.

Selon un aspect du procédé tel que défini ci-dessus, l'étape (a) de réaction d'un alcool de nature variable de formule (II) et d'un substrat de formule (III) comportant un motif oxétane et au moins une fonction hydroxyle est accompagnée d'un catalyseur 1 tel qu'un acide de Brönsted et/ou un acide de Lewis. Plus particulièrement, le catalyseur 1 est choisi parmi les acides sulfurique, sulfonique, chlorohydrique, nitrique, phosphorique, méthanesulfonique, p-toluènesulfonique, trifluoromethanesulfonique, le trifluorure de bore, le trichlorure d'aluminium ou le tétrachlorure d'étain.

Selon un autre aspect du procédé tel que défini ci-dessus, il comprend en outre l'étape :
- (b) réaction d'alcoxylation du composé de formule (I₁) telle que définie précédemment, accompagnée d'un catalyseur 2 et d'un oxyde d'alkylène ou un mélange d'oxydes d'alkylène ou d'un carbonate d'alkylène ou un mélange de carbonates d'alkylène. Le catalyseur 2 est un catalyseur basique tel que la potasse, la soude, le méthylate de sodium ou de potassium, le tertiobutylate de sodium ou de potassium, ou une base de Lewis comme la triphénylphosphine, ou un catalyseur de coordination comme par exemple des complexes organométalliques à base de Cobalt et/ou de Zinc, ou un acide de Lewis comme le trifluorure de bore, le trichlorure d'aluminium ou le tétrachlorure d'étain. L'oxyde d'alkylène ou le mélange d'oxydes d'alkylène utilisés dans de telles réactions d'alcoxylation sont les oxydes d'éthylène, de propylène ou encore de butylène. Compte-tenu du caractère hydrophobe des enchaînements propoxy- ou butoxy-, l'utilisation d'oxyde d'éthylène sera privilégiée.

Lors de cette nouvelle étape (b) optionnelle, les réactions d'alcoxylation modulent la Balance Hydrophile Lipophile (HLB) des structures de formule (I) décrites précédemment. Cette amphiphilie a conduit à valoriser ces nouvelles molécules en tant qu'agents tensioactifs ayant des propriétés tensiométriques, mouillantes, moussantes et émulsionnantes supérieures aux propriétés des agents connus.

L'invention a donc aussi pour objet l'utilisation d'un composé de formule (I) telle que définie précédemment, comme agent tensioactif non ionique et plus particulièrement, comme agent moussant, agent émulsionnant, agent mouillant, agent dispersant ou agent détergent. Une composition comprenant un mélange d'au moins un composé de formule (1), (I₁), (I₂) et (II) est également un objet de la présente invention.

L'invention a aussi pour objet une formulation détergente ou dégraissante caractérisée en ce qu'elle comprend comme agent tensioactif, au moins un composé de formule (I) telle que définie précédemment et/ou une composition comprenant un mélange d'au moins un composé de formule (I), (I₁), (I₂) et (II).

L'invention a enfin pour objet une formulation cosmétique ou pharmaceutique caractérisée en ce qu'elle comprend comme agent tensioactif, un composé de formule (I) telle que définie précédemment et/ou une composition comprenant un mélange d'au moins un composé de formule (I), (I₁), (I₂) et (II).

Par formulation cosmétique ou pharmaceutique on entend les formulations qui sont destinées à un usage topique, ou bien encore dans tout type de support destiné à être mis en contact avec la peau (papier, lingette, textile, dispositif transdermique, etc.). Ces formulations peuvent être appliquées sur la peau, sur les cheveux, sur le cuir chevelu et sur les muqueuses, et se présentent notamment sous la forme d'une solution aqueuse ou huileuse, d'une émulsion ou d'une micro-émulsion du type eau dans huile (E/H) ou huile-dans-eau (H/E), d'une émulsion multiple de type eau-dans-huile-dans-eau (E/H/E) ou huile-dans-eau-dans-huile (H/E/H), d'un gel, d'un savon ou d'un syndet, d'un baume, d'une hydro-dispersion, d'une pommade, d'une crème, d'une mousse, d'un aérosol ou encore sous forme anhydre comme une poudre. Ces formulations peuvent être utilisées comme laits nettoyants ou démaquillants, comme lotions nettoyantes ou démaquillantes, comme gels moussants pour le visage ou pour le corps, comme shampooings ou après-shampooings, comme bains moussants, comme crèmes de soin corporel, comme crèmes ou lotions pour le traitement du cuir chevelu.

De façon générale ces formulations comportent, en plus des composés de formule (I) et/ou des compositions comprenant un mélange d'au moins un composé de formule (I), (I₁), (I₂) et (II), des excipients et ou des principes actifs habituellement mis en oeuvre dans le domaine des formulations à usage topique, en particulier cosmétiques, dermocosmétiques, pharmaceutiques ou dermopharmaceutiques, tels que des épaississants, des gélifiants, des stabilisants, des composés filmogènes, des solvants et co-solvants, des agents hydrotropes, des agents plastifiants, des matières grasses, des huiles, des agents émulsionnants et co-émulsionnants, des agents opacificants, des agents nacrants, des agents surgraissants, des séquestrants, des agents chélatants, des antioxydants, des parfums, des conservateurs, des agents conditionneurs, des agents blanchissants destinés à la décoloration des poils et de la peau, des principes actifs destinés à apporter une action traitante vis à vis de la peau ou des cheveux, des filtres solaires, des charges minérales ou des pigments, des particules procurant un effet visuel ou destinées à l'encapsulation d'actifs, des particules exfoliantes, des agents de texture, des azurants optiques, des repellents pour les insectes.

Parmi les polymères épaississants et/ou émulsionnant utilisés dans les formulations cosmétiques ou pharmaceutiques de la présente invention, il y a par exemple, les homopolymères ou copolymères de l'acide acrylique ou de dérivés de l'acide acrylique, les homopolymères ou copolymères de l'acide méthacrylique ou de dérivés de l'acide méthacrylique, les homopolymères ou copolymères de l'acrylamide, les homopolymères ou copolymères de dérivés de l'acrylamide, les homopolymères ou copolymères de l'acide acrylamidométhyl propanesulfonique, les homopolymères ou copolymères de monomères vinyliques, les homopolymères ou copolymères de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes. Parmi les polymères de type polyélectrolytes, pouvant être mis en jeu dans la production d'une phase aqueuse gélifiée apte à être utilisée dans la préparation d'émulsions EIH, H/E, E/H/E ou H/E/H, ou d'un gel aqueux contenant les extraits de Potentille objets de la présente invention, il y a par exemple, les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800, SIMULGEL™ A, SIMULGEL™ EPG, SIMULGEL™ INS, SIMULGEL™ FL, SEPIGEL™ 501, SEPIGEL™ 502, SEPIPLUS™ 250, SEPIPLUS™ 265, SEPIPLUS™ 400, SEPINOV™ EMT 10, CARBOPOL™, ULTREZ™ 10, ACULYN™ , PEMULEN™ TR1, PEMULEN™ TR2, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA SP et STABILEZE™ 06.

Comme exemples de tensioactifs épaississants et/ou gélifiants éventuellement présents dans la formulation cosmétique ou pharmaceutique objet de la présente invention, on peut citer :
- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120.
- les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples d'émulsionnants éventuellement présents dans la formulation cosmétique ou pharmaceutique objet , on peut citer :
- les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Comme exemples d'agents opacifiants et/ou nacrants éventuellement présents dans la formulation cosmétique ou pharmaceutique objet de la présente invention, on peut citer les palmitates ou les stéarates ou les hydroxystéarates de sodium ou de magnésium, les monostéarates ou distéarates d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Comme exemples d'huiles éventuellement présentes dans la formulation cosmétique ou pharamaceutique objet de la présente invention,
on peut citer :
- les huiles minérales telles que l'huile de paraffine, l'huile de vaseline, les isoparaffines ou les huiles blanches minérales ;
- les huiles d'origine animale, telles que le squalène ou le squalane,
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, les isoalcanes de synthèse comme l'isohexadécane, l'isododécane, les huiles perfluorées et
- les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse éventuellement présente dans la formulation cosmétique ou pharmaceutique objet de la présente invention, on peut citer les alcools gras ou les acides gras ; les cires telles que la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène, les cires de silicone ; les cires végétales ; les alcools gras et les acides gras solides à température ambiante ; les glycérides solides à température ambiante.

Comme tensioactifs moussants et/ou détergents éventuellement présents dans la formulation cosmétique ou pharmaceutique objet de la présente invention, on peut citer : les tensioactifs anioniques, cationiques, amphotères ou non ioniques topiquement acceptables habituellement utilisés dans ce domaine d'activité.

Parmi les tensioactifs anioniques utilisables dans la présente invention, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Parmi les tensioactifs amphotères utilisables dans la présente invention, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques utilisables dans la présente invention, on citera particulièrement les dérivés d'ammoniums quaternaires.

Parmi les tensioactifs non ioniques utilisables dans la présente invention, on citera particulièrement les alkylpolyglycosides, les dérivés d'huile de ricin, les polysorbates, les amides de coprah, les N-alkylamines, les oxydes d'amines.

Comme exemples de principe actif que l'on peut utiliser dans la formulation cosmétique ou pharmaceutique objet de la présente invention, on peut citer les composés ayant une action éclaircissante ou dépigmentante tels que par exemple l'arbutine, l'acide kojique, l'hydroquinone, l'acide ellagique, la vitamine C et ses dérivés, le magnésium ascorbyl phosphate, le SEPIWHITE™ MSH, le SEPICALM™ VG, les extraits de polyphénols, les extraits de raisin, les extraits de pin, les extraits de vin, les extraits d'olives, les extraits de marc, les extraits de jus de pomme, les protéines N-acylées, les peptides N-acylés, les acides aminés N-acylés, comme par exemple le N-lauroyl proline, le N-linoléyl lysine, le N-linoléyl leucine, le N-octanoyl glycine, le N-undécylénoyl phénylalanine, le N-palmitoyl proline, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, les dérivés du Rétinol, la vitamine E et ses dérivés, les minéraux, les enzymes, les co-enzymes, comme, le Coenzyme Q10 et ses dérivés, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action bronzante comme la dihydroxyacétone et/ou l'érythrulose, les actifs ayant une action amincissante comme la caféine ou ses dérivés, les actifs ayant une activité anti-microbienne ou une action purifiante vis à vis des peaux grasses tels que le DEEPALINE™ PVB, le LIPACIDE™ UG, les actifs ayant une propriété énergisante ou stimulante comme par exemple le SEPITONIC™ M3 ou le Physiogényl™, le panthénol et ses dérivés comme le SEPICAP™ MP, les actifs anti-age comme le SEPILIFT™ DPHP, la DEEPALINE™ PVB, le SEPIVINOL™, le SEPIVITAL™, les actifs hydratants comme le SEPICALM™ S, le SEPICALM™ VG et le SEPILIFT™ DPHP, l'AQUAXYL™, le PROTEOL™ SAV 50, les actifs anti-age, les actifs présentant une action de tenseur ou de lissage immédiat sur la peau comme le SESAFLASH™, les actifs " anti-photo vieillissement ", les actifs protecteurs de l'intégrité de la jonction dermo-épidermique, les actifs augmentant la synthèse des composants de la matrice extracellulaire, les actifs ayant une activité amincissante, raffermissante ou drainante comme la caféine, la théophylline, l'Adénosyl Mono Phosphate cyclique (AMPc), le thé vert, la sauge, le ginko biloba, le lierre, le marron d'inde, le bambou, le ruscus, le petit houx, la centella asiatica, la bruyère, l'ulmaine, le fucus, le romarin, la saule, les extraits de panais, des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés), les actifs présentant une action vis-à-vis des cellules souches, les actifs présentant une action sur l'épiderme, le derme, l'hypoderme et les annexes cutanés (poils, glandes sébacées, pores, ...), les actifs présentant une action vis à vis de la flore cutanée.

Comme filtre solaire que l'on peut incorporer dans la formulation cosmétique ou pharmaceutique objet de l'invention, on peut citer tous ceux figurant dans la directive cosmétique 76/768/CEE modifiée annexe VII.

Tous les composés de formules (I), (I₁), (II) et (III) sont à considérer sous toute forme stéréo-isomérique possible.

La partie expérimentale suivante illustre l'invention.

### Exemple 1 : Préparation de structures amphiphiles.

### Etape a) : Ouverture du dérivé oxétane par l'alcool :

L'ouverture du dérivé oxétane par l'alcool est réalisée en présence d'un catalyseur acide à une température comprise entre 80°C et 160°C préférentiellement à 120°C. La quantité de catalyseur introduite est comprise entre 0,1% et 1,0% par rapport à la totalité du milieu de départ. La stoechiométrie molaire en dérivé oxétane, par rapport à l'alcool utilisé, est comprise entre 0,5 et 4,0 équivalents. Le temps de réaction sera dépendant de l'alcool de départ mais compris entre 60 minutes et 400 minutes.

Expérimentalement, l'alcool est chargé puis séché sous vide à chaud. Après remise à pression atmosphérique et inertage à l'azote, le catalyseur acide est introduit et le dérivé oxétane est ajouté progressivement dans le milieu pendant une durée comprise entre 60 et 180 min. En fin de réaction, un suivi de la teneur en dérivé oxétane résiduel est établi et après refroidissement, le produit peut être conditionné tel quel ou neutralisé par une base.

### Etape b) : Alcoxylation :

Le catalyseur acide introduit lors de l'étape a) peut éventuellement être neutralisé préalablement. Cette neutralisation peut notamment être réalisée par plusieurs lavages successifs à 80°C avec une solution aqueuse d'hydrogénocarbonate de sodium.

Après séchage sous vide, l'introduction d'oxyde est effectuée entre 100°C et 150°C et préférentiellement à 125°C dans le cas d'oxyde d'éthylène, en présence de 0,15%, par rapport au produit final, de potasse par exemple. La quantité d'oxyde mise en jeu sera adaptée au degré d'alcoxylation ciblé.

En fin d'addition, un maintien à température et à pression constantes est opéré et le milieu réactionnel est finalement déminéralisé par traitement sur silicate de magnésium dans le but d'éliminer tous résidus catalytiques.

Le tableau 1 suivant présente les caractéristiques analytiques de différents exemples de structures amphiphiles obtenues selon les protocoles de préparation décrits ci-avant.

**-Tableau 1- Caractéristiques des produits de réaction « triméthylolpropane oxétane (R=Et) / alcool » avec ou sans oxyde d'éthylène (Z₁=Z₂=H). I_{A} signifie indice d'acide, I_{OH} signifie indice d'hydroxyle, TA signifie température ambiante.**

| Réf. | **Alcool de formule (II)** | Stoech. mol. TMPO | Degré d'éthox.(m) | Degré d'éthox. **(A+B)** | Aspect à TA | pH 1% *pH 10%* | Eau (%) | Pt fusion (°C) *Visco à 25°C (cPs)* | IA (mg KOH/g) | IOH (mg KOH/g) | TMPO rés. (%) | Alcool(s) rés. (%) |
|---|---|---|---|---|---|---|---|---|---|---|---|---|
| Q | Oxo C-10 | 2,6 éq. | 0 | 5 | Liquide | 4,4 | 0,11 | *1915* | 0,17 | 282,6 | <0,1 | 1,0 |
| R | Oxo C-10 | 2,6 éq. | 0 | 10 | Liquide | 5,5 | 0,14 | *866* | 0,13 | 207,0 | <0,1 | 0,3 |
| S | Oxo C-10 | 2.6 éq. | 0 | 20 | Liquide | 5,3 | 0,31 | *598* | 0,16 | 133,7 | <0,1 | <0,1 |
| T | Décanol | 0,5 éq. | 6 | 0 | Liquide | 3,3 | 0,24 | *68* | 3,95 | 175,9 | <0,02 | 1,1 |
| U | Décanol | 0,75 éq. | 6 | 0 | Liquide | 3,4 | 0,33 | *118* | 3,67 | 194,2 | < 0,02 | 1,4 |
| V | Décanol | 1 éq. | 6 | 0 | Liquide | 3,4 | 0,27 | *174* | 3,51 | 212,1 | < 0,02 | 1,1 |
| V1 | Décanol | 1,5 éq. | 6 | 0 | Liquide | 3,6 | 0,19 | *1344* | 3,13 | 243,5 | < 0,02 | 0,8 |

### Exemple 2 : Evaluation des propriétés émulsionnantes :

Les différents tests ci-après ont pour objectif de mettre en évidence le caractère tensioactif des dérivés listés dans le tableau 1. Pour chaque test, le choix des structures étudiées a été orienté en fonction de l'hydrosolubilité ainsi que de l'ordre de grandeur de la HLB de ces dernières.

### 1) Abaissement de la tension superficielle de l'eau :

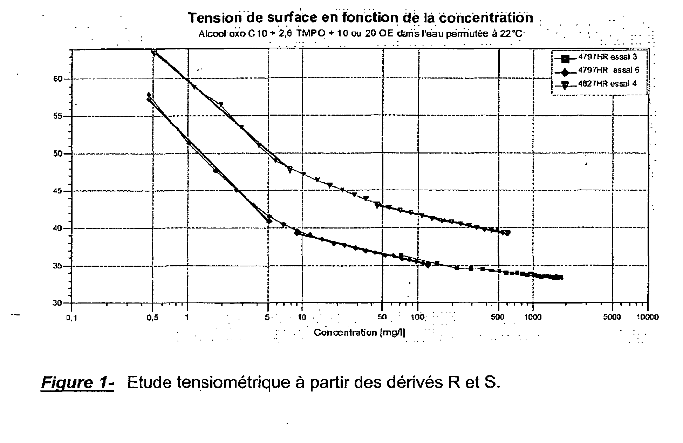

Les mesures de tensiométrie ont été menées à 20°C dans l'eau permutée à l'aide d'un tensiomètre K12™. Seuls les dérivés hydrosolubles R et S (tableau 1) ont été testés et les courbes d'abaissement de tension superficielle (exprimée en mN/m) de l'eau sont rassemblées sur la figure 1.

Les courbes de la figure 1 montrent clairement le caractère tensioactif des deux dérivés R et S. A une concentration égale à 1000 mg/l, les tensions de surface de l'eau sont respectivement égales à 33,9 et 39,3 mN/m.

### 2) Propriétés mouillantes :

Le mouillage d'un solide par un liquide correspond à l'étalement du liquide sur le solide. En diminuant la tension superficielle, les agents mouillants permettent un plus grand étalement du liquide.

### Principe de la mesure :

Le pouvoir mouillant a été déterminé sur coton écru, selon une méthodologie adaptée de celle décrite dans la norme NFT 73420, à deux températures, 20°C et 60°C et à une concentration en tensioactifs égale à 0,6 g/l dans l'eau permutée avec ou sans soude. Il est apprécié par la mesure de la durée de mouillage d'un disque de coton écru placé au sein d'une solution de tensioactifs à une concentration définie.

### Protocole expérimental :

On prépare 700 ml d'une solution d'essai comprenant 0.6 g/l de matière tensioactive dans de l'eau permutée. La solution d'essai est placée dans un bêcher thermostaté à la température souhaitée (20°C et 60°C, ± 1 °C).

La mesure expérimentale peut être réalisée au pH neutre de l'eau permutée ou en milieu basique en présence de soude.

Un disque de coton écru, répondant à la norme NFT 73-406 (30 mm de diamètre), est posé à la surface de la solution précédemment préparée à l'aide d'une pince d'immersion spécifique à ce test.

La durée de mouillage est déterminée expérimentalement à l'aide d'un chronomètre. L'instant t₀ correspond au moment où la partie inférieure du disque touche la solution et l'instant t_{final} correspond au moment où le disque s'enfonce par lui-même dans la solution. Dix mesures consécutives sont réalisées avec la même solution en prenant cependant soin de jeter les disques de coton utilisé après chaque mesure.

### Expression des résultats :

Le pouvoir mouillant est exprimé en seconde et il correspond à la moyenne des dix mesures réalisées.

### Résultats obtenus :

Les dérivés R à V (tableau 1) ont été testés et les temps d'imprégnation ont été comparés à ceux correspondant à un alcool oxo C10 éthoxylé et propoxylé commercialisé par la société SEPPIC sous l'appellation Simulsol™ NW 342 ou à des alcools oxo C-10 éthoxylés (tableau 2).

**-Tableau 2- Propriétés mouillantes des dérivés R à V.**

| Réf. Tensioactif | Temps en sec. à 20°C | Temps en sec. à 60°C |
|---|---|---|
| Simulsol™ NW 342 | 23 | 23 |
| Simulsol™ NW 342^{a)} | 30 | 50 |
| Simulsol™ NW 342^{b)} | 40 | 82 |
| Alcool oxo C-10 + 4,5 OE | 13 | 8 |
| Alcool oxo C-10 + 6,0 OE | 27 | 8 |
| R | 55 | 26 |
| S | 324 | 61 |
| T | 36 | 18 |
| U | 34 | 24 |
| V^{a)} | 97 | 88 |
| V^{b)} | 105 | 94 |
| U | 51 | 25 |

| | | |
|---|---|---|
| ^{a)}dans une solution de soude à 1 % dans l'eau permutée. ^{b)}dans une solution de soude à 2% dans l'eau permutée. | | |

Les pouvoirs mouillants des nouvelles structures de la présente invention sont très satisfaisants avec la particularité systématique de s'améliorer lorsque la température augmente.

### 3) Propriétés moussantes :

La formation de mousse, dispersion d'un volume important de gaz dans un faible volume de liquide, nécessite la présence d'agents tensioactifs qui s'adsorbent à l'interface eau-air.

### Principe de la mesure :

La détermination des propriétés moussantes par bullage d'azote permet d'évaluer les possibilités que peut avoir un tensioactif à former de la mousse. La mousse est formée par l'introduction d'un volume déterminé d'azote dans une solution de tensioactifs.

### Protocole expérimental :

50 ml d'une solution à 1 g/l de tensioactifs dans de l'eau permutée sont introduits dans une éprouvette graduée thermostatée de 250 ml. Les mesures ont été effectuées à 20 et 60°C et le pH lors des tests est celui des solutions de tensioactifs au départ. Un doigt pour distribution à gaz de porosité 3 (*réf* Coming Pyrex 853-1) est positionné de telle façon que l'extrémité de l'embout fritté se trouve à un centimètre du fond de l'éprouvette. Le débit d'azote est alors précisément réglé à 50 l/h et le barbotage est réalisé pendant 15 secondes. Après cette durée, l'arrivée de l'azote est coupée et l'expérimentateur note le volume de mousse initial ainsi que le volume de mousse après trente secondes, une minute, deux minutes et trente minutes. Trois essais ont été réalisés dans des éprouvettes différentes pour une même solution de tensioactif.

### Résultats obtenus :

Les dérivés R à V (tableau 1) ont été testés et les hauteurs de mousse relevées à différents temps ont été comparées à celles obtenues avec des alcools oxo C-10 éthoxylés (tableau 3).

**-Tableau 3- Propriétés moussantes des dérivés R à V.**

| Référence Tensioactif | ***Hauteur de mousse en cm au bout de 0*/*1*/*2*/*30 min.*** | |
|---|---|---|
| | 20°C | 60°C |
| **Alc. oxo C-10 + 4,5 OE** | 150/nd/115/10 | 155/nd/30/5 |
| **Alc. oxo C-10 + 6,0 OE** | 153 /nd/123/25 | 160/nd/103/15 |
| **R** | 148/120/110/20 | 155/115/45/0 |
| **S** | 150/120/110/18 | 175/135/135/0 |

| | ***Hauteur de mousse* en *cm au bout de 0*/*0,5*/ 1 / *2* / *30 min.*** | |
|---|---|---|
| | 20°C | 60°C |
| **T** | 178/nd/nd/130/25 | 180/nd/nd/130/0 |
| **U** | 148/nd/nd/113/13 | 168/135/83/20/0 |
| **V** | 140/100/65/38/10 | 125/20/13/5/0 |

Les pouvoirs moussants des nouvelles structures décrites sont comparables à ceux des références choisies et témoignent ainsi de leur caractère tensioactif.

### 4) Propriétés émulsionnantes Huile dans Eau :

Le pouvoir émulsionnant Huile dans Eau a été apprécié en faisant varier la nature du tensioactif (Q, S - tableau 1), la nature de l'huile ainsi que la proportion de chaque ingrédient. Différents protocoles d'émulsification ont été mis en oeuvre :

### x Emulsion 1 :

- Dispersion du tensioactif dans l'huile à 60°C,
- Ajout de la moitié d'eau pendant 1 min. à 1000 tr/min,
- Cisaillement par un agitateur cisaillant comprenant un système rotor-stator commercialisé par la société Silverson™ pendant 3 min. à 4000 tr/min,
- Ajout de l'autre moitié d'eau pendant 1 min. à 1000 tr/min,
- Cisaillement par un agitateur cisaillant comprenant un système rotor-stator commercialisé par la société Silverson™ pendant 1 min. à 4000 tr/min,
- Refroidissement à 30°C sous agitation avec une ancre à 200 tr/min.

### x Emulsions 2 et 3 :

- Homogénéisation à 60°C du tensioactif et de l'huile sous agitation avec une ancre à 200 tr/min,
- Ajout de l'eau et homogénéisation pendant 5 min,
- Cisaillement par un agitateur cisaillant comprenant un système rotor-stator commercialisé par la société Silverson™ pendant 3 min à 4000 tr/min,
- Refroidissement à 30°C sous agitation ancre à 200 tr/min.

### x Emulsion 4 :

- Mélange du tensioactif et de l'eau à 80°C,
- Ajout de l'huile et agitation à la spatule,
- Cisaillement par un agitateur cisaillant comprenant un système rotor-stator commercialisé par la société Silverson™ pendant 4 min. à 8000 tr/min,
- Refroidissement à température ambiante avec légère agitation ancre.

La stabilité de chaque émulsion a ensuite été suivie dans le temps à température ambiante et à 40°C (tableau 4).

**-Tableau 4- Stabilités des émulsions Huile dans Eau.**

| **Tensioactif (t.a)** (référence. émulsions) | Nature huile | Composition t.a/Huile/Eau | Observations | | |
|---|---|---|---|---|---|
| | | | t = 1 jour | t = variable | |
| | | | **Ambiante** | **Ambiante** | **40°C** |
| ***20% de Q* + *80% de Montane*^{™} *80^{a)}*** (Emulsion 1) | Huile de colza | 5/50/45 | Em. *^{b)}*: 100% | Em. *^{b)}*: 100% (8 jours) | Em. *^{b)}*: 100% (8 jours) |
| ***50% de Q* + *50% de S*** (Emulsion 2) | Exxsol D 100 | 2/20/78 | Em. *^{b)}*: 100% | Em. *^{b)}*∼ 85% (7 jours) | Em.*^{b)}*∼85% (7 jours) |
| ***25% de Q* + *75% de S*** (Emulsion 3) | Exxsol D 100 | 2/20/78 | Em. *^{b)}*∼ 98% | Em. *^{b)}*∼ 90% (7 jours) | Em. *^{b)}*∼ 90% (7 jours) |
| (Emulsion 4) | Triglycéride 5545 | 5/50/45 | Em. *^{b)}*∼ 100% | Em. *^{b)}*∼ 99% (30 jours) | Em. *^{b)}*∼ 50% (30 jours) |

| | | | | | |
|---|---|---|---|---|---|
| ^{a)}Monooléate de sorbitan commercialisé par Seppic. *^{b)}*Em : proportion de liquide se trouvant sous une forme d'émulsion homogène, évaluée à l'aide de graduation sur le contenant de stockage mis en stabilité. | | | | | |

Les résultats mettent en évidence des pouvoirs émulsionnants Huile dans Eau intéressants notamment lorsque les tensioactifs testés sont associés entre eux ou avec d'autres tensioactifs (le Montane™ 80 seul conduit à des émulsions Huile dans Eau non stables).

### 5) Propriétés émulsionnantes Eau dans Huile :

De manière similaire, le pouvoir émulsionnant Eau dans Huile a été apprécié en faisant varier la nature du tensioactif, la nature de l'huile ainsi que la proportion de chaque ingrédient. Le protocole d'émulsification mis en oeuvre est le suivant :
- Mélange du tensioactif et de l'huile à 80°C,
- Ajout de l'eau additivée de MgSO₄,
- Cisaillement par un agitateur cisaillant comprenant un système rotor-stator commercialisé par la société Silverson™ pendant 4 min. à 8000 tr/min,
- Refroidissement à température ambiante avec légère agitation ancre.

La stabilité de chaque émulsion a ensuite été suivie dans le temps à température ambiante et à 40°C.

Les résultats mettent en avant des pouvoirs émulsionnants bien supérieurs au Montane™ 60 (monostéarate de sorbitan commercialisé par Seppic) pris comme référence.

### Exemple 3 : Formulations

Dans les formules suivantes, les pourcentages sont exprimés en poids de la formulation.

### 3.1 Gel moussant visage

Formule

| | | |
|---|---|---|
| A | • Sodium lauryl sulfate | 5.9 % |
| | • Eau | q.s. 100 |
| B | • **Composé V selon l'invention** | 5.0 % |
| | • MONTALINE™ C40 | 5.0 % |
| | • SEPITONIC™ M3 | 1.0% |
| | • Parfum | 0.1 % |
| | • KATHON™ CG | 0.08% |
| C | • Acide lactique | 0.15 % |
| | • Chlorure de sodium | 0.8 % |
| D | • Colorant | 0.05% |
| E | • Chlorure de sodium | Qs |

### Mode opératoire

Préparer la phase A sous une hotte aspirante. Mélanger les ingrédients de la phase B en homogénéisant après chaque ajout. Verser ensuite A dans B. Ajouter C puis D. Ajuster la viscosité si nécessaire en ajoutant E (1.5% max).

### 3.2 Bain moussant pour enfants

Formule

| | | |
|---|---|---|
| A | • ORONAL™ LCG | 10.00 % |
| | • Composé **V selon l'invention** | 13.00 % |
| | • Parfum | 00.10% |
| | • SEPICIDE™ HB | 00.50 % |
| B | • Eau | 20.00% |
| | • CAPIGEL™ 98 | 04.50 % |
| C | • Eau | QSP 100% |
| | • SEPICIDE™ Cl | 00.30 % |
| | • Colorant | QS |
| | • Hydroxyde de sodium | QS |

### Mode opératoire

Mélanger ORONAL™ LCG avec le tensioactif selon l'invention, le parfum, et le conservateur. Diluer le CAPIGEL™ dans une partie d'eau et l'ajouter aux tensioactifs puis ajouter le reste d'eau. Ajouter le SEPICIDE™ CI, le colorant puis ajuster le pH à 7.2 environ.

### 3.3 Savon liquide pour les mains

Formule

| | | |
|---|---|---|
| A | • Composé **T selon l'invention** | 10.00 % |
| | • AMONYL™ 675SB | 10.00 % |
| | • Parfum/Fragrance | 00.30 % |
| | • SEPICIDE™ HB | 00.50 % |
| B | • Eau | QSP 100% |
| | • SEPICIDE™ Cl | 00.30 % |
| | • Chlorure de sodium | QS |

### Mode opératoire

Mélanger les ingrédients de la phase A puis ajouter la phase B.

### 3.4 Mousse nettoyante visage

Formule

| | | |
|---|---|---|
| A | • PROTEOL™ OAT | 3.00 % |
| | • Composé **W selon l'invention** | 10.00 % |
| | • SEPICIDE HB™ | 00.50 % |
| | • Parfum | 00.20 % |
| B | • Eau | QSP 100% |
| | • SEPICIDE™ Cl | 00.30 % |
| | • SEPITONIC M3 | 01.00 % |
| | • Tromethamine | QS |
| | • Colorant | QS |

### Mode opératoire

Solubiliser le parfum et le conservateur dans le mélange des tensioactifs (A). Ajouter l'eau puis les autres ingrédients successivement.

### 3.5 Lingettes nettoyantes

Formule

| | | |
|---|---|---|
| A | • **Composé V selon l'invention** | 02.00 % |
| | • AQUAXYL™ | 01.00 % |
| B | • SEPICIDE™ HB₂ | 00.50 % |
| | • Parfum | 00.05% |
| | • Hexylene glycol | 10.00 % |
| C | • Eau | Qsp 100 % |

### Mode opératoire

Mélanger les ingrédients de la phase B jusqu'à limpidité puis ajouter cette phase à la phase A. Ajouter C.

### 3.6 Savon liquide antibactérien

| | |
|---|---|
| **Composé V selon l'invention** | 15,00 % |
| Digluconate de chlorhexidine | 00,20 % |
| ORAMIDE™ DL 200 AF | 03,00 % |
| Eau | QSP100 |
| Parfum | 00,05 % |
| Colorant | QS |
| Chlorure de sodium | QS |

### Mode opératoire

Ajouter et mélanger les constituants dans l'ordre indiqué.

### 3.7 Shampooing conditionneur

| | | |
|---|---|---|
| A | • Cetyl trimethyl ammonium chloride | 03.50 % |
| | • Composé **V selon l'invention** | 25.00 % |
| | • Dimethicone copolyol | 01.00 % |
| | • Parfum | 00.50 % |
| | • AMONYL™ 380BA | 11.00 % |
| | • KATHON™ CG | 0.08 % |
| B | • Polyquatemium•10 | 00.30 % |
| | • Acide lactique | QS |
| | • Eau | QSP 100 % |
| | • Colorant | QS |
| | • Acide lactique | QS PH=6 |

### Mode opératoire

Préparer séparément la phase B: mélanger jusqu'à limpidité. Mélanger soigneusement les ingrédients dans l'ordre indiqué.

### 3.8 Crème de soin

| | |
|---|---|
| Cyclométhicone : | 10% |
| SIMULGEL EG : | 3,0% |
| Composé K selon l'invention : | 2% |
| Alcool stéarylique : | 1% |
| Alcool stéarique : | 0,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | q.s.p. 100% |

### 3.9 Lait solaire

Formule

| | | |
|---|---|---|
| A | Composé E selon l'invention : | 3,0% |
| | Huile de sésame : | 5,0% |
| | PARSOL™ MCX : | 5,0% |
| | Carraghénane λ : | 0,10% |
| B | Eau : | q.s.p. 100% |
| C | SIMULGEL EG : | 3,00% |
| D | Parfum : | q.s. |
| | Conservateur : | q.s. |

### Mode opératoire

Emulsionner B dans A à 75°C puis ajouter C vers 60°C, puis D vers 30°C et ajuster le pH si nécessaire.

### 3.10 Fond de teint hydratant et matifiant

Formule

| | | |
|---|---|---|
| A | eau : | 20,0% |
| | Butylène glycol : | 4,0% |
| | PEG-400 : | 4,0% |
| | PECOSIL™ PS100 : | 1,0% |
| | Hydroxyde de Sodium : | q.s. pH = 9 |
| | Dioxyde de titane : | 7,0% |
| | Talc : | 2,0% |
| | Oxyde de fer jaune : | 0,8% |
| | Oxyde de fer rouge : | 0,3% |
| | Oxyde de fer noir : | 0,05% |
| B | LANOL™ 99 : | 8% |
| | Caprylic capric triglycéride | 8% |
| | Composé G selon l'invention : | 5,00% |
| C | eau : | q.s.p. 100% |
| | MICROPEARL™ M305 : | 2,0% |
| | EDTA tétrasodé : | 0,05% |
| D | Cyclométhicone : | 4,0% |
| | Gomme de Xanthane : | 0,2% |
| | SIMULGEL NS : | 3,0% |
| E | SEPICIDE™ HB : | 0,5% |
| | SEPICIDE CI : | 03% |
| | Parfum : | 0,2% |

### Mode opératoire

Préparer à 80°C, les mélanges B + D et A + C, puis mélanger et émulsionner l'ensemble.
3.11 Lait corporel

| | |
|---|---|
| Composé E selon l'invention : | 3,5% |
| LANOL™ 37T : | 8,0% |
| SOLAGUM™ L : | 0,05% |
| Eau : | q.s.p. 100% |
| Benzophénone-3 : | 2,0% |
| Diméthicone 350cPs : | 0,05% |
| SIMULGEL EG : | 3,0% |
| Conservateur : | 0,2% |
| Parfum : | 0,4% |

3.12 Emulsion démaquillante à l'huile d'amandes douces

| | |
|---|---|
| Composé L selon l'invention : | 5% |
| Huile d'amandes douces : | 5% |
| Eau : | q.s.p. 100% |
| SIMULGEL EG : | 2,5% |
| Glycérine : | 5% |
| Conservateur : | 0,2% |
| Parfum: | 0,3% |

3.13 Crème hydratante pour peaux grasses

| | |
|---|---|
| Composé L selon l'invention : | 5% |
| Cétylstéaryloctanoate : | 8% |
| Palmitate d'octyle : | 2% |
| Eau : | q.s.p. 100% |
| SIMULGEL NS | 2,5% |
| MICROPEARL™ M100 : | 3,0% |
| Mucopolysaccharides : | 5% |
| SEPICIDETM HB : | 0,8% |
| Parfum : | 0,3% |

3.14 Crème aux AHA pour peaux sensibles

| | |
|---|---|
| Mélange de N-lauryl aminoacides : | 0,1% à 5% |
| Aspartate de magnésium et de potassium : | 0,002% à 0,5% |
| LANOL™ 99 : | 2% |
| Composé L selon l'invention : : | 5,0% |
| Eau : | q.s.p. 100% |
| SIMULGEL EG : | 3,0% |
| Acide gluconique : | 1,50% |
| Triéthanolamine (TEA) : | 0,9% |
| SEPICIDE™ HB : | 0,3% |
| SEPICIDE™ CI : | 0,2% |
| Parfum : | 0,4% |

3.15 Lait démaquillant

| | |
|---|---|
| Composé E selon l'invention : | 3% |
| PRIMOL™ 352 : | 8,0% |
| Huile d'amandes douces : | 2% |
| Eau : | q.s.p. 100% |
| SIMULGEL NS : | 3,0% |
| Conservateur : | 0,2% |

3.16 Lait solaire

| | |
|---|---|
| Composé E selon l'invention :: | 3,5% |
| LANOL™ 37T : | 10,0% |
| PARSOL™ MCX : | 5,0% |
| EUSOLEX™ 4360 : | 2,0% |
| Eau : | q.s.p. 100% |
| SIMULGEL 600: | 3,0% |
| Conservateur : | 0,2% |
| Parfum : | 0,4% |

3.17 Crème aux AHA

| | |
|---|---|
| Composé L selon l'invention : | 5,0% |
| DEEPALINE™ PVB: | 1,05% |
| LANOL™ 99 : | 10,0% |
| eau : | q.s.p. 100% |
| Acide gluconique : | 1,5% |
| TEA (triéthanolamine) : | 0,9% |
| SIMULGEL EG: | 3,0% |
| Parfum: | 0,4% |
| SEPICIDE™ HB: | 0,2% |
| SEPICIDE™ CI: | 0,4% |

3.18 Emulsion bronzante sans soleil

| | |
|---|---|
| LANOL™ 99 : | 15% |
| Composé L selon l'invention : | 5,0% |
| PARSOL™ MCX : | 3,0% |
| Eau : | q.s.p. 100% |
| Dihydroxyacétone : | 5,0% |
| Phosphate monosodique : | 0,2% |
| SIMULGEL EG : | 2,5% |
| Parfum : | 0,3% |
| SEPICIDE™ HB : | 0,8% |
| Hydroxyde de sodium : | q.s. pH=5. |

3.19 Crème de soin

| | |
|---|---|
| Cyclométhicone : | 10% |
| SIMULGEL NS: | 3,0% |
| Composé L selon l'invention : | 4,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | qsp 100% |

### 3.20 Lait corporel

Formule

| | | |
|---|---|---|
| A | Composé E selon l'invention : | 3,0% |
| | Triheptonate de glycérol : | 10,0% |
| | B Eau : | q.s.p. 100% |
| C | SIMULGEL NS: | 3,0% |
| D | Parfum : | q.s. |
| | Conservateur : | q.s. |

### Mode opératoire

Fondre A à environ 75°C. Emulsionner B dans A à 75°C puis ajouter C vers 60°C, puis D.
3.21 Crème aux AHA

| | |
|---|---|
| Composé K selon l'invention : | 5,0% |
| DEEPALINE™ PVB : | 1,05% |
| LANOL™ 99 : | 10,0% |
| Eau : | q.s.p. 100% |
| Acide gluconique : | 1,5% |
| TEA (triéthanolamine) : | 0,9% |
| SIMULGEL 600 | 1,5% |
| Parfum : | 0,4% |
| SEPICIDE™ HB : | 0,2% |
| SEPICIDE™ CI : | 0,4% |

3.22 Lait démaquillant

| | |
|---|---|
| SIMULSOL™ 165 : | 4% |
| Composé G selon l'invention : | 1% |
| Caprylate-caprate triglyceride : | 15% |
| PECOSIL™ DCT : | 1% |
| Eau déminéralisée : | qs |
| CAPIGEL™ 98 : | 0,5% |
| SIMULGEL EG | 3% |
| PROTEOL™ APL : | 2% |
| Hydroxyde de sodium : | qsp pH = 7 |

3.23 Crème solaire

| | |
|---|---|
| SIMULSOL™ 165 : | 3% |
| Composé G selon l'invention : | 2% |
| Benzoate C12-C15 : | 8% |
| PECOSIL™ PS 100 : | 2% |
| Diméthicone : | 2% |
| Cyclométhicone : | 5% |
| Para-méthoxy cinnamate d'octyle : | 6% |
| Benzophénone-3 : | 4% |
| Oxyde de Titane : | 8% |
| Gomme xanthane : | 0,2% |
| Butylèneglycol : | 5% |
| Eau déminéralisée : | qsp 100% |
| SIMULGEL NS : | 2,5% |
| Conservateur, parfum : | qs |

3.24 Crème vitaminée

| | |
|---|---|
| SIMULSOL™ 165 : | 5% |
| Composé G selon l'invention : | 1% |
| Caprylic/capric triglycérides : | 20% |
| Palmitate de vitamine A : | 0,2% |
| Acétate de vitamine E : | 1% |
| MICROPEARL™ M 305 : | 1,5% |
| SIMULGEL EG | 3% |
| Eau | qsp 100% |
| Conservateur, parfum | qs |

3.25 Gel solaire et autobronzant

| | |
|---|---|
| Composé E selon l'invention : | 3,0% |
| Triheptanoate de glycéryle : | 10,0% |
| DEEPALINE™ PVB : | 1,05% |
| SIMULGEL 600 | 2,2% |
| Eau : | qs 100% |
| Dihydroxyacétone : | 5% |
| Parfum : | 0,1% |
| SEPICIDE™ HB : | 0,3% |
| SEPICIDE™ CI : | 0,1% |
| PARSOL™ MCX : | 4,0% |

3.26 Crème autobronzante aux α-hydroxy acides

| | |
|---|---|
| Composé G selon l'invention : | 5,0% |
| DEEPALINE™ PVB : | 1,05% |
| Lanol™ 99 : | 10,0% |
| Eau: | qs 100% |
| Acide gluconique : | 1,5% |
| Dihydroxyacétone : | 3% |
| Triéthanolamine : | 0,9% |
| SIMULGEL NS | 3,0% |
| Parfum : | 0,4% |
| SEPICIDE™ HB : | 0,2% |
| SEPICIDE™ CI : | 0,4% |

3.27 Crème autobronzante aux α-hydroxy acides pour peau sensible

| | |
|---|---|
| Mélange de N-lauroyl aminoacides : | 0,1% à 5% |
| Aspartate de magnésium et de potassium : | 0,002% à 0,5% |
| Composé H selon l'invention : | 5,0% |
| Lanol™ 99 : | 2,0% |
| Eau : | qs 100% |
| Acide lactique : | 1,5% |
| Dihydroxyacétone : | 3,5% |
| Triéthanolamine : | 0,9% |
| SIMULGEL NS : | 1,5% |
| Parfum : | 0,4% |
| SEPICIDE™ HB : | 0,3% |
| SEPICIDE™ CI : | 0,2% |

3.28 Dégraissant industriel

| | |
|---|---|
| Composé U selon l'invention : | 5% |
| Bicarbonate de sodium : | 3,5% |
| Hydrogénocarbonate de sodium: | 3,5% |
| EDTA sel disodique | 5% |
| POLYMERE AC : | 0,2% |
| SEPPIC 1050 : | 1,5% |
| TRITON H 66 : | 3,5% |
| SIMULSOL PG 711 : | 2,0% |
| Eau : | qs 100% |

Le SEPITONIC™ M3, mélange d'aspartate de magnésium, de gluconate de zinc et de gluconate de cuivre, est un actif énergisant commercialisé par la société SEPPIC.

Le Polyquaternium™ 10 est un sel d'ammonium quaternaire d'une hydroxyéthylcellulose, commercialisé par la société AMERCHOL sour l'appelation UCARE POLYMER JR - 400.

Le KATHON™ CG, mélange de méthylchloroisothiazolinone et de méthylisothiazolinone, est un agent conservateur commercialisé par la société ROHM & HAAS.

L'ORONAL™ LCG, mélange de PEG-40 glyceryl cocoate et de cocethsulfate de sodium, est un agent moussant commercialisé par la société SEPPIC.

L'AMONYL™ 675SB est une Cocoamidopropyl hydroxy sultaïne, commercialisée par la société SEPPIC.

Le SEPICIDE™ HB, un mélange de phénoxyéthanol, de méthylparaben, d'éthylparaben, de propylparaben et de butylparaben, est un agent conservateur commercialisé par la société SEPPIC.

Le CAPIGEL™ 98 est un copolymère d'acrylates commercialisé par la société SEPPIC.

Le SEPICIDE™ Cl, imidazoline urée, est un agent conservateur commercialisé par la société SEPPIC.

Le SEPICIDE™ HB2, un mélange de phénoxyéthanol, de méthylparaben, d'éthylparaben, de propylparaben, de butylparaben et d'isobutylparaben, est un agent conservateur commercialisé par la société SEPPIC.

Le PROTEOL™ OAT est un mélange de N-lauryl aminoacides obtenus par hydrolyse totale de protéine d'avoine tel que décrit dans WO 94/26694 et commercialisé par la société SEPPIC.

La MONTALINE™ C40 est un sel de chlorure de Cocamidopropyl betaïnamide de Monoéthanolamine.

L'AQUAXYL™ est un actif hydratant, comprenant un mélange de Xylitylpolyglucosides, d'anhydroxylitol et de xylitol, commercialisé par la société SEPPIC.

L'ORAMIDE™ DL 200 AF est une Cocamide de Di Ethanol Amide, commercialisée par la société SEPPIC.

L'AMONYL™ 380BA est une Cocamidopropyl betaïne, commercialisée par la société SEPPIC.

LANOL™ 99 est de l'isononanoate d'isononyle commercialisé par la société SEPPIC.

Le MICROPEARL™ M 100 est une poudre ultra fine au toucher très doux et à action matifiante commercialisée par la société MATSUMO.

Le MICROPEARL^{™} M 305 est une poudre ultra fine au toucher très doux et à action matifiante commercialisée par la société MATSUMO.

Le SEPICIDE™ CI, imidazolidine urée, est un agent conservateur commercialisé par la société SEPPIC.

PEMULEN™ TR1 est un polymère acrylique commercialisé par GOODRICH.

Le SIMULSOL™ 165 est du stéarate de glycérol auto-émulsionnable commercialisée par la société SEPPIC.

Le PARSOL™ MCX est du para-méthoxy cinnamate d'octyle ; commercialisé par la société GIVAUDAN.

Le LANOL™ 37T est du triheptanoate de glycérol, commercialisé par la société SEPPIC.

Le SOLAGUM™ L est un carraghénane commercialisé par la société SEPPIC.

L' EUSOLEX™ 4360 est un filtre solaire commercialisé par la société MERCK.

La DEEPALINE™ PVB, est un hydrolysât de protéines de blé acylé commercialisé par la société SEPPIC.

Le PROTEOL™ APL est un tensioactif moussant, commercialisée par la société SEPPIC.

Le PRIMOL™ 352 est une huile minérale commercialisée par la société EXXON.

Le PECOSIL™ DCT est du sodium Dimethicone PEG-7 Acetyl Methyltaurate commercialisé par la société PHOENIX.

Le PECOSIL™PS 100 est du Dimethicone PEG-7 commercialisé par la société PHOENIX.

Le SIMULGEL™ EG est un latex inverse auto-inversible de copolymères tel que ceux décrits dans la publication internationale WO 99/36445 (dénomination INCI : Sodium acrylate/Sodium acryloyldimethyl taurate copolymer et Isohexadecane et Polysorbate 80) commercialisé par la société SEPPIC.

Le SIMULGEL™ NS est un latex inverse auto-inversible de copolymère tel que ceux décrits dans la publication intemationale WO 99/36445 (dénomination INCI : hydroxyethylacrylate/Sodium acryloyldimethyl taurate copolymer et squalane et Polysorbate 60) commercialisé par la société SEPPIC.

Le SIMULGEL™ 600 est un latex inverse auto-inversible (dénomination INCI : Acrylamide/Sodium acryloyldimethyl taurate copolymer / Isohexadecane /Polysorbate 80) commercialisé par la société SEPPIC.

Le POLYMERE AC est un polyacrylate de sodium utilise comme agent dispersant.

Le TRITON H 66 est phosphate de m,p Crésol éthoxylé utilisé comme agent hydrotrope et solubilisant.

Le SIMULSOL PG 711 est un dérivé propoxylé de l'alcool Stéarylique utilisé comme agent anti-moussant.

Le SEPPIC 1050 est une composition comprenant du citrate de monoéthanolamine et du tartrate de monoéthanolamine, utilisée comme agent anti-corrosion.

## Revendications

1. Composé de formule (I) : 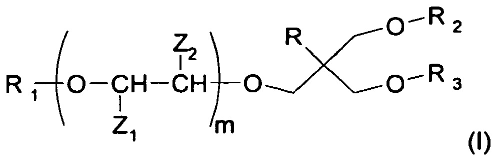 dans laquelle :
- R₁ représente un radical aliphatique saturé ou insaturé, linéaire ou ramifié, comportant de 7 à 24 atomes de carbone, éventuellement substitué avec un ou plusieurs groupes hydroxyle,
- R représente un radical alkyle linéaire ou ramifié comportant de un à huit atomes de carbone éventuellement substitué par un radical hydroxyle,
- m représente un nombre supérieur ou égal à 0 et inférieur ou égal à 150,
- Z₁ et Z₂ identiques ou différents sont choisis parmi H, CH₃, CH₂-CH₃,
- R₂ et R₃ identiques ou différents représentent soit un atome d'hydrogène, soit un radical monovalent de formule (a) : 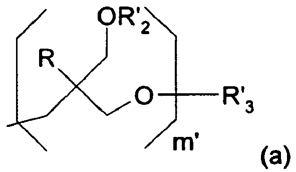 dans laquelle :
- m' représente un nombre inférieur ou égal à 10,
- R'₂ représente soit un atome d'hydrogène, soit un radical monovalent de formule (b) : 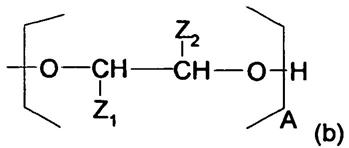 dans laquelle :
■ Z₁ et Z₂ sont tels que définis ci-dessus,
■ A représente un nombre entier supérieur ou égal à 0 et inférieur ou égal à 50,
- R'₃ identique à R'₂ ou différent de R'₂ représente soit un atome d'hydrogène, soit un radical monovalent de formule (c) : 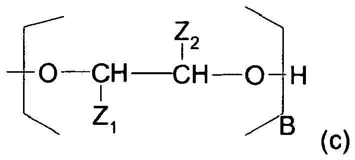 dans laquelle :
■ Z₁ et Z₂ sont tels que définis ci-dessus,
■ B représente un nombre entier supérieur ou égal à 0 et inférieur ou égal à 50,
avec A + B supérieur ou égal à 0 et inférieur ou égal à 50 et m + A + B ≠ 0 ; **caractérisé en ce que** R₂ représente un atome d'hydrogène et R₃ représente un radical de formule (a') : 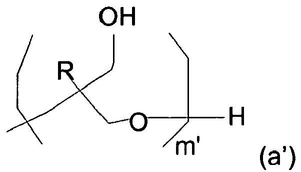 correspondant à la formule (a) dans laquelle R'₂ et R'₃ représentent chacun un atome d'hydrogène.

2. Composé de formule (I'₀) correspondant à la formule (I) telle que définie à la revendication 1 dans laquelle R₃ représente un radical de formule (a') : 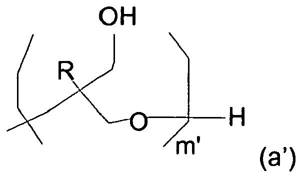 correspondant à la formule (a) dans laquelle R'₂ et R'₃ représentent chacun un atome d'hydrogène.

3. Composé de formule (I₁) telle que définie à la revendication 1 dans laquelle R'₂ et R'₃ représentent un atome d'hydrogène.

4. Composé de formule (I₂) telle que définie à la revendication 1 dans laquelle l'un ou l'autre parmi R'₂ et R'₃ ne représente pas un atome d'hydrogène.

5. Composé de formule (I) telle que définie à l'une des revendications précédentes dans laquelle A et B sont identiques.

6. Composé de formule (I) telle que définie à l'une des revendications précédentes dans laquelle R est choisi parmi les radicaux méthyle, éthyle, propyle, butyle, pentyle, hexyle, heptyle, octyle, méthylol.

7. Composition comprenant au moins un composé de formule (II) : 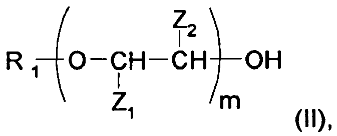 dans laquelle R₁, Z₁ et Z₂ sont tels que définis ci-dessus,
et un mélange d'au moins un composé choisi parmi les composés de formule (I), (I₁), (I₂) telle que définie à l'une des revendications 1 à 6.

8. Procédé de préparation d'un composé de formule (I₁) correspondant à la formule (I) telle que définie à l'une des revendications 1 à 7, dans laquelle R'₂ et R'₃ représentent un atome d'hydrogène, comprenant l'étape :
- a) réaction d'au moins un alcool de formule (II): 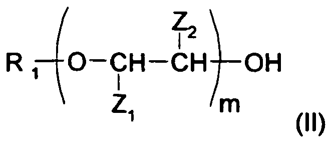 dans laquelle R₁, Z₁ et Z₂ sont tels que définis dans la formule (I) ;
et d'au moins un substrat, comportant un motif oxétane et au moins une fonction hydroxyle, de formule (III): 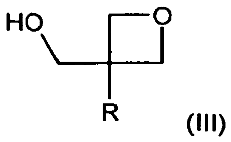 dans laquelle R est tel que défini dans la formule (I) ;
et si nécessaire, l'étape :
- b) réaction d'alcoxylation du composé de formule (I₁), accompagnée d'un catalyseur 2 et d'un oxyde, pour la préparation d'un composé de formule (I₂), telle que définie à la revendication 5.

9. Procédé tel que défini à la revendication 8, **caractérisé en ce que** ledit au moins un substrat de formule (III) est choisi parmi le triméthyloléthane-oxétane, le triméthylolpropane- oxétane, le triméthylolbutane-oxétane, le triméthylolpentaneoxétane, le triméthylolhexane-oxétane, le triméthylolheptane-oxétane, le triméthyloloctane- oxétane, le triméthylolnonane-oxétane, le pentaérythritol-oxétane.

10. Utilisation d'au moins un composé de formule (I) telle que définie à l'une des revendications 1 à 6 et/ ou d'une composition telle que définie à la revendication 7, comme agent tensioactif ou, comme agent moussant, agent émulsionnant, agent mouillant, agent dispersant ou agent détergent.

11. Formulation cosmétique ou pharmaceutique **caractérisée en ce qu'**elle comprend comme agent tensioactif, au moins un composé de formule (I) telle que définie à l'une des revendications 1 à 6 et/ou une composition telle que définie à la revendication 7.

12. Formulation détergente ou dégraissante **caractérisée en ce qu'**elle comprend comme agent tensioactif, au moins un composé de formule (I) telle que définie à l'une des revendications 1 à 6 et/ou une composition telle que définie à la revendication 7.

## Claims

1. Compound of formula (I) : 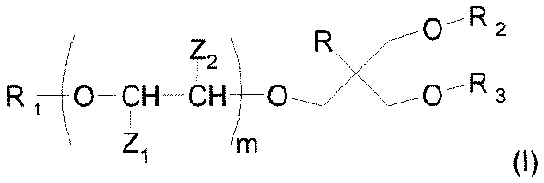 in which:
- R₁ represents a linear or branched and saturated or unsaturated aliphatic radical comprising from 7 to 24 carbon atoms, optionally substituted by one or more hydroxyl groups,
- R represents a linear or branched alkyl radical comprising from 1 to 8 carbon atoms, optionally substituted by a hydroxyl radical,
- m represents a number greater than or equal to 0 and less than or equal to 150,
- Z₁ and Z₂, which are identical or different, are chosen from H, CH₃ or CH₂-CH₃,
- R₂ and R₃, which are identical or different, represent either a hydrogen atom or a monovalent radical of formula (a) : 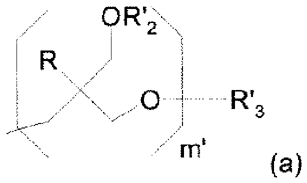
in which:
- m' represents a number less than or equal to 10,
- R'₂ represents either a hydrogen atom or a monovalent radical of formula (b): 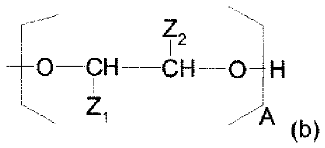
in which:
■ Z₁ and Z₂ are as defined above,
■ A represents an integer greater than or equal to 0 and less than or equal to 50,
- R'₃, which is identical to R'₂ or different from R'₂, represents either a hydrogen atom or a monovalent radical of formula (c): 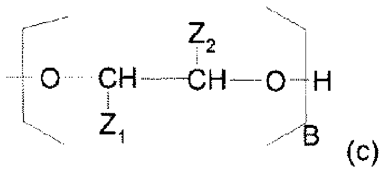
in which:
■ Z₁ and Z₂ are as defined above,
■ B represents an integer greater than or equal to 0 and less than or equal to 50,
with A + B greater than or equal to 0 and less than or equal to 50 and m + A + B ≠ 0; **characterized in that**
R₂ represents a hydrogen atom and R₃ represents a radical of formula (a'): 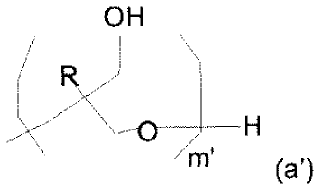 corresponding to the formula (a) in which R'₂ and R'₃ each represent a hydrogen atom.

2. Compound of formula (I'₀) corresponding to the formula (I) as defined in Claim 1 in which R₃ represents a radical of formula (a'): 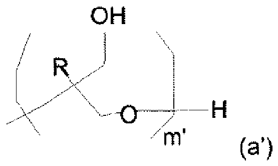 corresponding to the formula (a) in which R'₂ and R'₃ each represent a hydrogen atom.

3. Compound of formula (I₁) as defined in Claim 1 in which R'₂ and R'₃ represent a hydrogen atom.

4. Compound of formula (I₂) as defined in Claim 1 in which one or other among R'₂ and R'₃ does not represent a hydrogen atom.

5. Compound of formula (I) as defined in one of the preceding claims, in which A and B are identical.

6. Compound of formula (I) as defined in one of the preceding claims, in which R is chosen from the methyl, ethyl, propyl, butyl, pentyl, hexyl, heptyl, octyl or methylol radicals.

7. Composition comprising at least one compound of formula (II) : 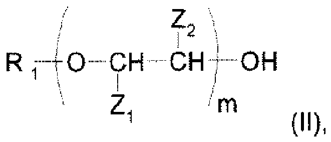 in which R₁, Z₁ and Z₂ are as defined above,
and a mixture of at least one compound chosen from the compounds of formula (I), (I₁) or (I₂) as defined in one of Claims 1 to 6.

8. Process for the preparation of a compound of formula (I₁) corresponding to the formula (I) as defined in one of Claims 1 to 7 in which R'₂ and R'₃ represent a hydrogen atom, comprising the stage:
- a) reaction of at least one alcohol of formula (II) : 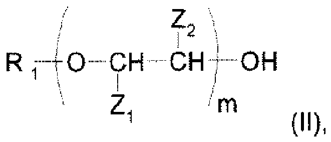 in which R₁, Z₁ and Z₂ are as defined in the formula (I);
and at least one substrate, comprising an oxetane unit and at least one hydroxyl functional group, of formula (III) : 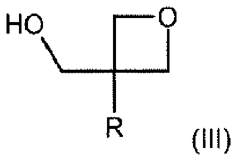 in which R is as defined in the formula (I);
and, if necessary, the stage:
- b) alkoxylation reaction of the compound of formula (I₁), accompanied by a catalyst 2 and by an oxide, for the preparation of a compound of formula (I₂) as defined in Claim 4.

9. Process as defined in Claim 8, **characterized in that** said at least one substrate of formula (III) is chosen from trimethylolethane-oxetane, trimethylolpropane-oxetane, trimethylolbutane-oxetane, trimethylolpentane-oxetane, trimethylolhexane-oxetane, trimethylolheptane-oxetane, trimethyloloctane-oxetane, trimethylolnonane-oxetane or pentaerythritol-oxetane.

10. Use of at least one compound of formula (I) as defined in one of Claims 1 to 6 and/or of a composition as defined in Claim 7 as surface-active agent or as foaming agent, emulsifying agent, wetting agent, dispersing agent or detergent agent.

11. Cosmetic or pharmaceutical formulation, **characterized in that** it comprises, as surface-active agent, at least one compound of formula (I) as defined in one of Claims 1 to 6 and/or a composition as defined in Claim 7.

12. Detergent or degreasing formulation, **characterized in that** it comprises, as surface-active agent, at least one compound of formula (I) as defined in one of Claims 1 to 6 and/or a composition as defined in Claim 7.

## Patentansprüche

1. Verbindung der Formel (I): 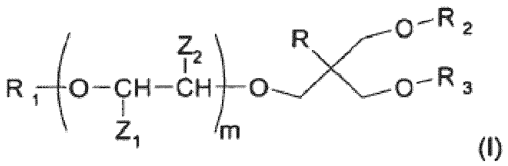 in der:
- R₁ für einen linearen oder verzweigten, gesättigten oder ungesättigten aliphatischen Rest mit 7 bis 24 Kohlenstoffatomen, der gegebenenfalls durch eine oder mehrere Hydroxylgruppen substituiert ist, steht,
- R für einen linearen oder verzweigten Alkylrest mit einem bis acht Kohlenstoffatomen der gegebenenfalls durch einen Hydroxylrest substituiert ist, steht,
- m für eine Zahl größer gleich 0 und kleiner gleich 150 steht,
- Z₁ und Z₂ gleich oder verschieden sind und aus H, CH₃ und CH₂-CH₃ ausgewählt sind,
- R₂ und R₃ gleich oder verschieden sind und entweder für ein Wasserstoffatom oder einen einwertigen Rest der Formel (a) steht: 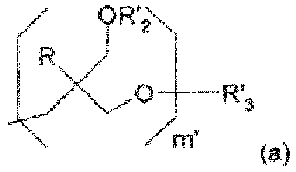
in der:
- m' für eine Zahl kleiner gleich 10 steht,
- R'₂ entweder für ein Wasserstoffatom oder einen einwertigen Rest der Formel (b) steht: 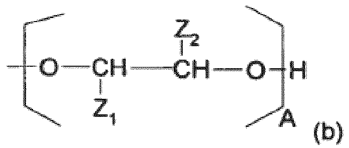
in der:
■ Z₁ und Z₂ wie oben definiert sind,
■ A für eine ganze Zahl größer gleich 0 und kleiner gleich 50 steht,
- R'₃ mit R'₂ identisch oder von R'₂ verschieden ist und entweder für ein Wasserstoffatom oder einen einwertigen Rest der Formel (c) steht: 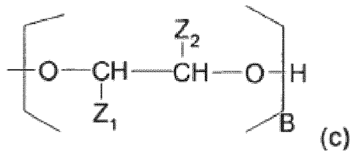
in der:
■ Z₁ und Z₂ wie oben definiert sind,
■ B für eine ganze Zahl größer gleich 0 und kleiner gleich 50 steht,
wobei A + B größer gleich 0 und kleiner gleich 50 ist und m + A + B ≠ 0 ist;
**dadurch gekennzeichnet, dass** R₂ für ein Wasserstoffatom steht und R₃ für einen Rest der Formel (a') steht: 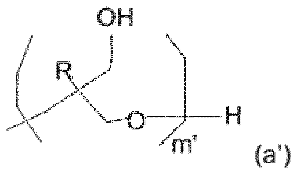 die der Formel (a), in der R'₂ und R'₃ jeweils für ein Wasserstoffatom stehen, entspricht.

2. Verbindung der Formel (I'₀) entsprechend der Formel (I) nach Anspruch 1, in der R₃ für einen Rest der Formel (a') steht: 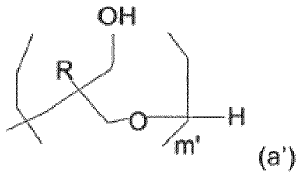 die der Formel (a), in der R'₂ und R'₃ jeweils für ein Wasserstoffatom stehen, entspricht.

3. Verbindung der Formel (I₁) nach Anspruch 1, in der R'₂ und R'₃ für ein Wasserstoffatom stehen.

4. Verbindung der Formel (I₂) nach Anspruch 1, in der R'₂ oder R'₃ nicht für ein Wasserstoffatom steht.

5. Verbindung der Formel (I) nach einem der vorhergehenden Ansprüche, in der A und B gleich sind.

6. Verbindung der Formel (I) nach einem der vorhergehenden Ansprüche, in der R aus den Methyl-, Ethyl-, Propyl-, Butyl-, Pentyl-, Hexyl-, Heptyl-, Octyl- oder Methylolresten ausgewählt ist.

7. Zusammensetzung, umfassend mindestens eine Verbindung der Formel (II): 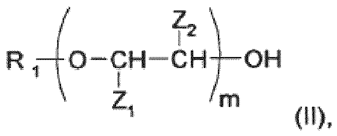 in der R₁, Z₁ und Z₂ wie oben definiert sind,
und eine Mischung von mindestens einer unter den Verbindungen der Formel (I), (I₁) und (I₂) nach einem der Ansprüche 1 bis 6 ausgewählten Verbindung.

8. Verfahren zur Herstellung einer Verbindung der Formel (I₁) entsprechend der Formel (I) nach einem der Ansprüche 1 bis 7, in der R'₂ und R'₃ für ein Wasserstoffatom stehen, bei dem man:
- a) mindestens einen Alkohol der Formel (II): 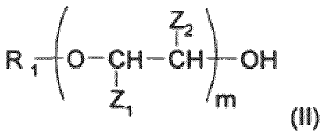 in der R₁, Z₁ und Z₂ wie in der Formel (I) definiert sind;
und mindestens ein Substrat, das eine Oxetaneinheit und mindestens eine Hydroxylfunktion enthält, der Formel (III) : 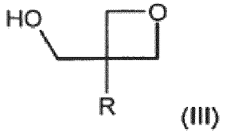 in der R wie in der Formel (I) definiert ist; umsetzt;
und gegebenenfalls:
- b) die Verbindung der Formel (I₁) zur Herstellung einer Verbindung der Formel (I₂) nach Anspruch 4 in Begleitung eines Katalysators 2 und eines Oxids alkoxyliert.

9. Verfahren nach Anspruch 8, **dadurch gekennzeichnet, dass** man das mindestens eine Substrat der Formel (III) aus Trimethylolethan-oxetan, Trimethylolpropan-oxetan, Trimethylolbutan-oxetan, Trimethylolpentan-oxetan, Trimethylolhexan-oxetan, Trimethylolheptan-oxetan, Trimethyloloctan-oxetan, Trimethylolnonan-oxetan und Pentaerythritol-oxetan auswählt.

10. Verwendung mindestens einer Verbindung der Formel (I) nach einem der Ansprüche 1 bis 6 und/oder einer Zusammensetzung nach Anspruch 7 als Tensid oder als Schaummittel, Emulgator, Netzmittel, Dispergiermittel oder waschaktives Mittel.

11. Kosmetische oder pharmazeutische Formulierung, **dadurch gekennzeichnet, dass** sie als Tensid eine Verbindung der Formel (I) nach einem der Ansprüche 1 bis 6 und/oder eine Zusammensetzung nach Anspruch 7 umfasst.

12. Waschaktive oder entfettende Formulierung, **dadurch gekennzeichnet, dass** sie als Tensid eine Verbindung der Formel (I) nach einem der Ansprüche 1 bis 6 und/oder eine Zusammensetzung nach Anspruch 7 umfasst.
